# EUROPÄISCHE PATENTSCHRIFT

(11) **EP 0 490 816 B1**
(45) Veröffentlichungstag und Bekanntmachung des Hinweises auf die Patenterteilung: **09.07.1997**
(21) Anmeldenummer: 91810940.6
(22) Anmeldetag: 03.12.1991
(51) Int. Cl.: C07D 249/08, C07D 233/56, C07D 249/04, C07D 257/04, C07D 239/26, C07D 213/26, C07D 405/06, A61K 31/395

(54) **Fluorverbindungen als Aromatase-Inhibitoren**
Fluoro-compounds and their use as aromatase inhibitors
Dérivés fluorés et leur utilisation comme inhibiteurs de aromatase

(30) Priorität: 12.12.1990 CH 3923/90
(43) Veröffentlichungstag der Anmeldung: 17.06.1992
(73) Patentinhaber: Novartis AG, 4058 Basel (CH)
(72) Erfinder: Lang, Marc, Dr., F-68200 Mülhausen (FR); Differding, Edmond, Dr., B-1348 Louvain-la Neuve (BE); Stanek, Jaroslav, Dr., CH-4144 Arlesheim (CH)

(56) Entgegenhaltungen:
- EP-A- 0 118 245
- EP-A- 0 163 606
- EP-A- 0 236 940
- EP-A- 0 299 683
- EP-A- 0 299 684
- EP-A- 0 337 929
- US-A- 5 006 543
- J. STEROID BIOCHEM. MOLEC. BIOL., Band 37, Nr. 6, PROCEEDINGS OF THE 2ND INTERNATIONAL EORTC SYMPOSIUM, Rotterdam, 9. - 11. April 1990, Seiten 1021- 1027, Pergamon Press plc, Oxford, GB; A.S. BHATNAGAR et al.: "Highly selective inhibition of estrogen biosynthesis by CGS 20267, a new non-steroidal aromatase inhibitor"
- J. Med. Chem., 1987, 30, S. 1359-1365
- J. Med. Chem., 1990, 33, S. 416-429

## Beschreibung

Die Erfindung betrifft Verbindungen der Formel I, 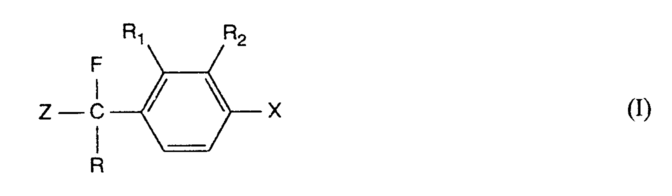 worin Z 1-Imidazolyl, 1-(1,2,4-Triazolyl), 1-(1,3,4-Triazolyl), 1-(1,2,3-Triazolyl), 1-Tetrazolyl oder 2-Tetrazolyl bedeutet; R₁ und R₂ unabhängig voneinander Wasserstoff oder Niederalkyl bedeuten; oder R₁ und R₂ zusammen für 1,4-Butylen oder eine Benzogruppe stehen; R für 4-Cyanophenyl oder 4-Bromophenyl steht; und X Cyano bedeutet; und Salze davon, Verfahren zur Herstellung dieser Verbindungen, pharmazeutische Präparate, die diese Verbindungen enthalten, die Verwendung dieser Verbindungen zur Herstellung pharmazeutischer Präparate.

Die Europäischen Patentanmeldungen EP 0 236 940 und EP 0 337 929, das US-Patent US 5,006,543,J. Steroid Biochem. Mol. Biol. 37, 1021-27 (1990), J. Med. Chem. 30, 1359-65 (1987) und J. Med. Chem. 33, 416-29 (1990) nennen jeweils Aromatasehemmer, die sich jedoch von den Verbindungen der vorliegenden Erfindung strukturell unterscheiden und nicht deren positive Eigenschaften aufweisen.

Die Verbindungen der Formel I, welche ein asymmetrisches Kohlenstoffatom enthalten, können jeweils als Racemat oder als R- oder S-Enantiomer vorliegen. Die Erfindung bezieht sich auf alle diese Formen sowie z.B. auch auf Diastereomere und Gemische davon, die auftreten können, wenn zwei oder mehr asymmetrische Zentren im Molekül vorhanden sind, sowie auf geometrische Isomere, z.B. cis- und trans-Isomere, wenn das Molekül eine Doppelbindung enthält.

Die vor- und nachstehend verwendeten Allgemeinbegriffe haben im Rahmen der vorliegenden Anmeldung vorzugsweise die folgenden Bedeutungen:

Das Präfix "Nieder" bezeichnet einen Rest bis und mit höchstens 7, insbesondere bis und mit höchstens 4, und vor allen Dingen 1 oder 2 Kohlenstoffatomen.

Niederalkyl ist z.B. n-Propyl, Isopropyl, n-Butyl, Isobutyl, sek-Butyl, tert-Butyl, n-Pentyl, Neopentyl, n-Hexyl oder n-Heptyl, vorzugsweise Ethyl und vor allem Methyl.

Bedeuten R₁ und R₂ zusammen in Verbindungen der Formel I C₄-Alkylen, so bilden diese Reste zusammen mit dem Benzolring ein 1,2,3,4-Tetrahydronaphthalin-Gerüst. Bedeuten R₁ und R₂ zusammen eine Benzogruppe, so bilden diese Reste zusammen mit dem Benzolring ein Naphthalingerüst.

Salze von erfindungsgemässen Verbindungen sind in erster Linie pharmazeutisch verwendbare, nicht-toxische Salze. Beispielsweise können Verbindungen der Formel I mit basischen Gruppen Säureadditionssalze bilden, z.B. mit anorganischen Säuren, wie Salzsäure, Schwefelsäure oder Phosphorsäure, oder mit geeigneten organischen Carbon- oder Sulfonsäuren, z.B. Essigsäure, Fumarsäure oder Methansulfonsäure, oder mit Aminosäuren, wie Arginin oder Lysin.

Zur Isolierung oder Reinigung können auch pharmazeutisch ungeeignete Salze, z.B. Pikrate oder Perchlorate, Verwendung finden. Zur therapeutischen Anwendung gelangen nur die pharmazeutisch verwendbaren, nicht-toxischen Salze, die deshalb bevorzugt sind.

Die erfindungsgemässen Verbindungen der Formel I weisen wertvolle, insbesondere pharmakologisch verwendbare, Eigenschaften auf. Insbesondere hemmen sie selektiv das Enzym Aromatase bei Säugern einschliesslich Menschen. Dadurch wird die metabolische Umwandlung von Androgenen zu Oestrogenen gehemmt. Die Verbindungen der Formel I sind daher z.B. zur Behandlung Oestrogen-abhängiger Krankheiten, einschliesslich Oestrogen-abhängigem Brustkrebs, insbesondere bei postmenopausalen Frauen, geeignet. Ferner sind sie z.B. nützlich bei der Behandlung von Gynäkomastie, d.h. männlicher Brustentwicklung, weil die Aromatisierung der Steroide gehemmt wird.

Diese Wirkungen können durch in vitro-Versuche oder in vivo-Versuche, vorzugsweise an Säugetieren, z.B. Meerschweinchen, Mäusen, Ratten, Katzen, Hunden oder Affen, nachgewiesen werden. Die angewandte Dosierung liegt z.B. in einem Bereich von etwa 0,001 und 10 mg/kg, vorzugsweise zwischen 0,001 und 1 mg/kg.

Die in vitro-Hemmung der Aromataseaktivität kann z.B. mit der Methode, die in J. Biol. Chem. 249, 5364 (1974) beschrieben ist, gezeigt werden. Ferner können IC₅₀-Werte für die Aromatasehemmung z.B. in vitro aus enzymkinetischen Studien, die die Hemmung der Umwandlung von 4-¹⁴C-Androstendion zu 4-¹⁴C-Oestron in menschlichen plazentalen Mikrosomen betreffen, erhalten werden. Die IC₅₀-Werte der erfindungsgemässen Verbindungen liegen minimal etwa bei 10⁻⁹ M.

In vivo kann die Aromatasehemmung z.B. durch die Unterdrückung des ovarialen Oestrogengehalts weiblicher Ratten gezeigt werden, die zunächst mit Stutenserumgonadotropin und 2 Tage später mit menschlichem Choriongonadotropin injiziert werden, am folgenden Tag p.o. mit einer Verbindung der Erfindung behandelt werden und 1 h später mit Androstendion. Eine weitere Möglichkeit zur in vivo Bestimmung der Aromatasehemmung wird im folgenden beschrieben: Androstendion (30 mg/kg subkutan) wird allein bzw. zusammen mit einer Verbindung der Erfindung (oral oder subkutan) 4 Tage lang an nicht geschlechtsreife weibliche Ratten verabreicht. Nach der vierten Anwendung werden die Ratten getötet, die Uteri isoliert und gewogen. Die Aromatasehemmung wird bestimmt durch das Ausmass, in dem die durch die Verabreichung von Androstendion allein verursachte Uterushypertrophie durch die gleichzeitige Verabreichung der erfindungsgemässen Verbindung unterdrückt bzw. verringert wird. Die minimale effektive Dosis der erfindungsgemässen Verbindungen bei den in vivo Tests liegt etwa zwischen 0,001 und 1 mg/kg.

Die Antitumorwirkung, insbesondere bei Oestrogen-abhängigen Tumoren, kann in vivo z.B. bei DMBA-induzierten Mammatumoren an weiblichen Sprague-Dawley-Ratten gezeigt werden [vgl. Proc. Soc. Exp. Biol. Med. 160, 296-301 (1979)]. Die Anwendung von erfindungsgemässen Verbindungen bewirkt eine Regression der Tumoren und unterdrückt weiterhin das Auftreten neuer Tumoren bei täglichen Dosen ab etwa 1 mg/kg p.o.

Darüberhinaus haben die Verbindungen der Formel I keine Hemmwirkung auf die Spaltung der Cholesterin-Seitenkette und induzieren keine adrenale Hypertrophie, was durch endokrine Organuntersuchungen gezeigt wird.

Aufgrund ihrer pharmakologischen Eigenschaften als äusserst selektive Inhibitoren des Enzyms Aromatase sind die Verbindungen der Formel I z.B. zur Behandlung Oestrogenabhängiger Krankheiten, wie Brusttumoren (Brustkarzinoma), Endometriosis, vorzeitigen Wehen oder endometrialen Tumoren bei Frauen oder Gynäkomastie bei Männern, geeignet.

Bevorzugt betrifft die Erfindung die Verbindungen der Formel I, worin Z 1-Imidazolyl, 1-(1,2,4-Triazolyl), 1-(1,2,3-Triazolyl), 1-Tetrazolyl oder 2-Tetrazolyl bedeutet; R₁ und R₂ für Wasserstoff stehen; R 4-Cyanophenyl oder 4-Bromophenyl bedeutet; und X für Cyano steht; und pharmazeutisch verwendbare Salze davon.

Als Untergruppen aus einer Gruppe von Verbindungen der Formel I sind jeweils hervorzuheben:
(a) Verbindungen der Formel I, worin Z 1-(1,2,4-Triazolyl) bedeutet; und (b) Verbindungen der Formel I, worin R₁ und R₂ Wasserstoff bedeuten.

Die Erfindung betrifft vor allem die in den Beispielen beschriebenen spezifischen Verbindungen und pharmazeutisch verwendbare Salze davon.

Die Verbindungen der Formel I können in an sich bekannter Weise hergestellt werden, indem man z.B.
(a) eine Verbindung der Formel II, 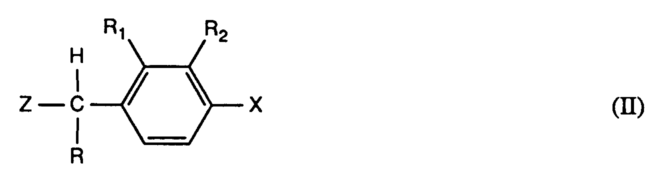 worin Z, R, R₁, R₂ und X wie unter Formel I definiert sind, mit einem Fluorierungsmittel umsetzt, oder
(b) zur Herstellung von Verbindungen der Formel I, worin Z über ein Ringkohlenstoffatom gebunden ist, eine Verbindung der Formel III, 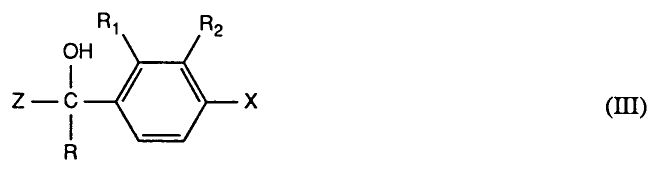 worin Z, R, R₁, R₂ und X wie unter Formel I definiert sind, mit einem Fluorierungsmittel umsetzt, oder
(c) eine Verbindung der Formel IIIa, 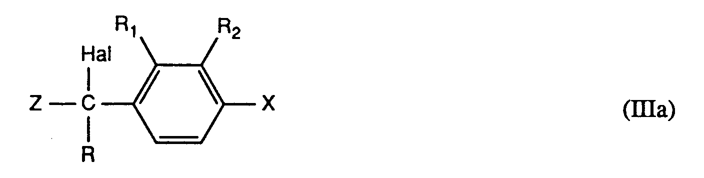 worin Hal Chlor, Brom oder Iod bedeutet und Z, R, R₁, R₂ und X wie unter Formel I definiert sind, mit einem Fluorierungsmittel umsetzt, oder
(d) eine Verbindung der Formel IV, 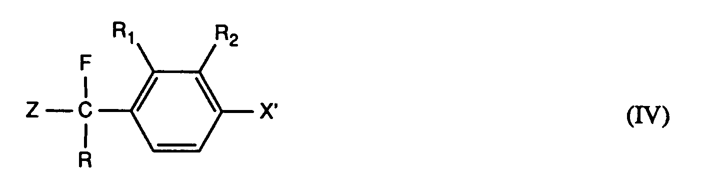 worin Z, R, R₁ und R₂ wie unter Formel I definiert sind und X′ eine in einen Rest X überführbare Gruppe bedeutet, die Gruppe X′ in den Rest X überführt, und/oder eine erhaltene Verbindung der Formel I in eine andere Verbindung der Formel I umwandelt, und/oder ein erhaltenes Salz in die freie Verbindung oder in ein anderes Salz umwandelt, und/oder eine erhaltene freie Verbindung der Formel I in ein Salz umwandelt, und/oder ein erhaltenes Gemisch von isomeren Verbindungen der Formel I in die einzelnen Isomeren auftrennt.

In der folgenden Beschreibung der Verfahren haben die Symbole Z, R, R₁, R₂ und X jeweils die unter Formel I angegebene Bedeutung, sofern nichts anderes angegeben ist.

Verfahren (a): Das Verfahren (a) wird z.B. derart durchgeführt, dass man eine Verbindung der Formel II durch Umsetzung mit einer starken Base, z.B. einem Alkalimetall-diisopropylamid, etwa Lithium-, Natrium- oder Kaliumdiisopropylamid, einer Hexamethyldisilazanbase, etwa Kalium-, Natrium- oder Lithiumhexamethyldisilazan, oder einer Alkalimetallbase, etwa sek.-, tert.- oder n-Butyllithium, in das entsprechende Carbanion überführt und letzteres mit einem elektrophilen Fluorierungsmittel umsetzt.

Als elektrophile Fluorierungsmittel sind z.B. zu nennen [s. z.B. auch L. German und S. Zemskov (Hrsg.), New Fluorinating Agents in Organic Synthesis, Springer, Berlin etc. 1989]:
1. Fluorierungsmittel mit F-Halogen-Bindung, z.B. F₂ [s. z.B. Chem. Rev. 86,997 (1986)] oder Perchlorylfluorid [FClO₃, s. z.B. J. Amer. Chem. Soc. 80, 6533 (1958)].
2. Reagenzien mit F-Sauerstoff-Bindung, z.B. Trifluormethylhypofluorit [CF₃OF, s. z.B Isr. J. Chem. 17, 60 (1978)], Acetylhypofluorit [CH₃COOF, s. z.B. J. Org. Chem. 46, 4629 (1981)] oder Trifluoracetylhypofluorit [CF₃COOF, s. z.B. J. Org. Chem. 45, 4122 (1980)].
3. Reagenzien mit F-Stickstoff-Bindung, z.B. N-F-Sulfonamide [s. z.B. J. Amer. Chem. Soc. 106, 452 (1984) oder J. Fluor. Chem. 46, 297 (1990)], insbesondere N-Fluor-3,3-dimethyl-2,3-dihydro-1,2-benzothiazol-1,1-dioxid [s. z.B. Helv. Chim. Acta 72, 1248 (1989)], N-F-Sulfonimide [s. z.B. J. Amer. Chem. Soc. 109, 7194 (1987)], N-F-Pyridiniumderivate [s. z.B. J. Amer. Chem. Soc. 112, 8563 (1990)], insbesondere N-Fluor-2,4,6-trimethylpyridinium-trifluormethylsulfonat, N-F-Perfluorpiperidine [s. z.B. Chem. and Industry (London) 1964, 1864], N-F-Quinuclidiniumsalze [s. z.B. J. Fluor. Chem. 41, 297 (1988) oder J. Chem. Soc. Perkin Trans. I 1988, 2805], N-F-Pyridone [s. z.B. J. Fluor. Chem. 26, 43 (1984)] oder N-F-Amide oder -Lactame [s. z.B. J. Org. Chem. 55, 3373 (1990)].
4. Reagenzien mit F-Edelgas-Bindung, z.B. Xenondifluorid (XeF₂) [s. z.B. M. Zupan in: S. Patai und Z. Rappoport (Hrsg.), The Chemistry of Functional Groups, Supplement D, Part 2, Wiley, Chichester etc. 1983].
5. Enantioselektive Fluorierungsmittel, z.B. von Campher abgeleitete N-Fluorsultame [s. z.B. Tetrahedron Lett. 29, 6087 (1988)], die insbesondere zur stereoselektiven Fluorierung von Verbindungen der Formel II, bei welchen das zentrale Kohlenstoffatom chiral ist, geeignet sind.

Die Ausgangsverbindungen der Formel II werden z.B. wie in EP-A-236 940 beschrieben oder in analoger Weise dazu hergestellt (siehe auch EP-Patentanmeldung Nr. 90810515.8).

Zum Beispiel werden Verbindungen der Formel II, worin Z die für Verbindungen der Formel I genannten Bedeutungen hat, hergestellt, indem man eine Verbindung der Formel V, 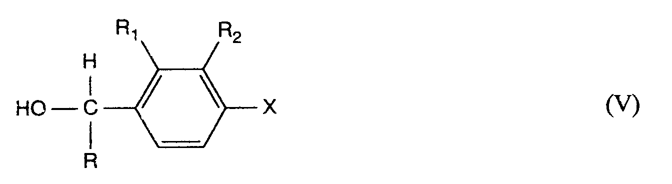 oder insbesondere ein reaktives verestertes Derivat davon, z.B. ein Halogenderivat oder ein Niederalkyl- oder Aryl-sulfonyloxyderivat wie Methylsulfonyloxy oder p-Toluolsulfonyloxy, mit einer Verbindung der Formel VI,

Z-H (VI)

oder einem N-geschützten Derivat davon, z.B. durch Triniederalkylsilyl, Niederalkanoyl, N,N-Diniederaalkylcarbamoyl oder Triphenylmethyl, umsetzt.

Die Ausgangsverbindungen der Formel V werden z.B. durch Umsetzung einer metallierten Verbindung Va, 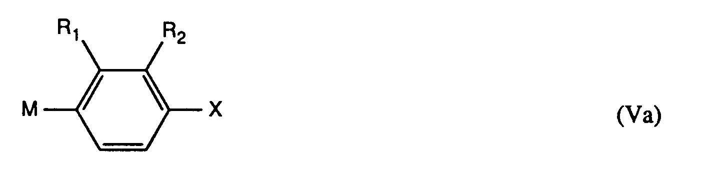 worin M z.B. für Halogenmagnesium, Kupfer(I)lithium oder insbesondere Li steht, mit einem Aldehyd R-CHO hergestellt.

Weiterhin können Verbindungen der Formel II z.B. dadurch hergestellt werden, dass man eine Verbindung der Formel VII 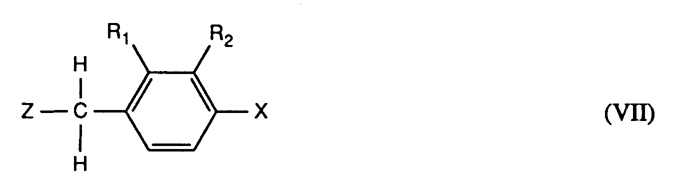 unter basischen Bedingungen mit einem reaktiven funktionellen Derivat des Restes R, z.B. einem Halogen-, Niederalkyl- oder Aryl-sulfonyloxyderivat davon, umsetzt.

Verbindungen der Formel II, worin Z über ein Ringkohlenstoffatom gebundenes Heteroaryl bedeutet, können ferner z.B. hergestellt werden, indem man eine Verbindung der Formel VIII, 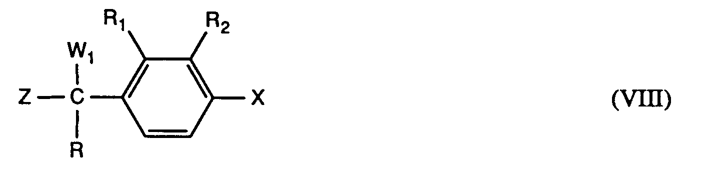 worin W₁ Hydroxy oder Halogen bedeutet, reduziert bzw. dehalogeniert. Die Reduktion von W₁ = OH kann z.B. mit Zinn(II)chlorid oder Eisessig/wässrige Iodwasserstoffsäure durchgeführt werden. Die Dehalogenierung von W₁ = Halogen, z.B. Chlor, kann beispielsweise mit Zink/Essigsäure, Tributylzinnhydrid oder Aluminiumamalgam erfolgen.

Die Ausgangsverbindungen der Formel VIII mit W₁ = OH werden z.B. durch Reaktion eines Aldehyds bzw. Ketons der Formel IX 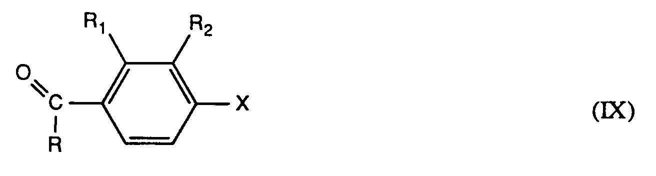 mit einer Verbindung X,

Z-W₂ (X)

worin W₂ Wasserstoff oder eine an dem entsprechenden Ringkohlenstoff sitzende Schutzgruppe oder Abgangsgruppe, etwa Triniederalkylsilyl oder Halogen, bedeutet, hergestellt.

Die Ausgangsverbindungen der Formel VIII mit W₁ = Halogen werden vorteilhaft aus den entsprechenden Verbindungen der Formel VIII mit W₁ = OH hergestellt. Dazu werden letztere mit einem Halogenierungsmittel, z.B. Thionylchlorid, umgesetzt.

Verbindungen der Formel II können ferner z.B. dadurch hergestellt werden, dass man eine Verbindung der Formel XI 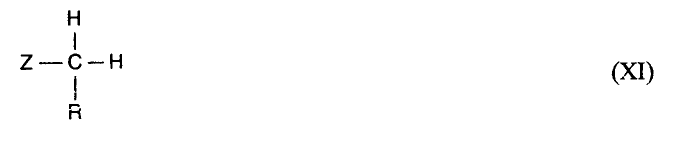 im basischen Medium mit einer Verbindung der Formel XII, 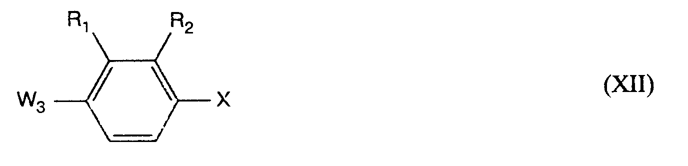 worin W₃ eine Abgangsgruppe bedeutet, z.B. Halogen, Niederalkyl- oder Arylsulfonyloxy und insbesondere Fluor, umsetzt.

Eine weitere Möglichkeit zur Herstellung von Verbindungen der Formel II, insbesondere solchen, in denen Z über ein Ringstickstoffatom gebunden ist, besteht darin, in einer Verbindung der Formel XIII, 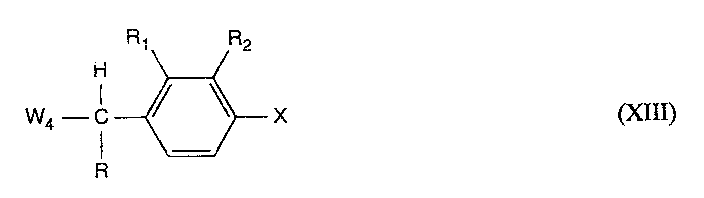 worin W₄ einen in Z überführbaren Rest, z.B. Isocyano, Amino, Azido oder Hydrazino bedeutet, den Rest W₄ durch Reaktion mit entsprechenden ringbildenden Reagenzien in Z zu überführen.

Ferner ist es z.B. möglich, Verbindungen der Formel II aus entsprechenden Verbindungen der Formel XIV, 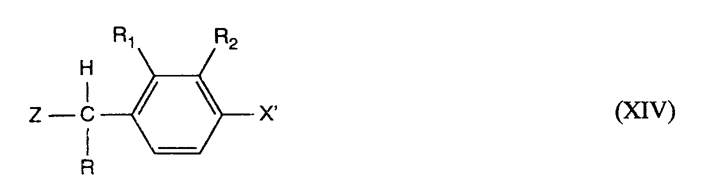 worin X' eine in einen Rest X überführbare Gruppe [siehe unter Verfahren (d)] bedeutet, durch Umwandlung der Gruppe X' in X herzustellen.

Die Ausgangsverbindungen der Formel XIV werden z.B. analog den oben angegebenen Verfahren zur Herstellung von Verbindungen der Formel II hergestellt, wobei in den entsprechenden Umsetzungen anstelle des Restes X ein Rest X' eingesetzt wird.

Die Verbindungen der Formel II sind wertvolle Zwischenprodukte zur Herstellung der therapeutisch wertvollen Fluorverbindungen der Formel I.Insbesondere die Verbindung 4-[α-(4-Cyanophenyl)-1-(1,2,3-triazolyl)methyl]-benzonitril, sowie Salze davon, ist hier hervorzuheben.

Darüberhinaus weist diese Verbindung wertvolle pharmakologisch verwendbare Eigenschaften auf. Sie wirkt in gleicher Weise wie die Verbindungen der Formel I als Aromatasehemmer. Ihre Aktivität liegt in der gleichen Grössenordnung wie die der Verbindungen der Formel I (s. oben). Daher ist sie auch nützlich z.B. zur Behandlung der oben bei den Verbindungen der Formel I angegebenen Krankheiten. Die Verbindung kann - wie die Verbindungen der Formel I - in pharmazeutische Präparate eingearbeitet und zur Behandlung von Krankheiten, die auf die Hemmung der Aromatase ansprechen, sowie (zur Herstellung von pharmazeutischen Präparaten) für die Tumorbehandlung verwendet werden.

Verfahren (b): Zur Fluorierung der Hydroxyverbindungen III sind z.B. die folgenden Fluorierungsmittel geeignet: sekundäres Amino-schwefeltrifluoride, z.B. Piperidino-schwefeltrifluorid oder Diethylamino-schwefeltrifluorid ["DAST", s. z.B. J. Org. Chem. 40, 574 (1975)]; SF₄ (s. z.B. Organic Reactions 34, 319, Wiley, New York etc. 1985), z.B. im System SF₄/HF; Fluoralkylamin-Reagenzien der Formel (R')₂N-CF₂-R mit R' = z.B. Ethyl und R = z.B. CHFCl [s. z.B. Organic Reactions 21, 158, Wiley, New York etc. 1974]; α-Fluor-enamine [s. z.B. Tetrahedron Lett. 30, 3077 (1989)]; oder Triethylamin/HF [s. z.B. Tetrahedron Lett. 31, 6527 (1990)].

Die Ausgangsverbindungen der Formel III werden z.B. durch Umsetzung eines Aldehyds bzw. Ketons der Formel IX (s. oben) mit einer Verbindung der Formel X, Z-W₂, worin W₂ Wasserstoff oder eine an dem entsprechenden Ringkohlenstoffatom sitzende Schutzgruppe oder Abgangsgruppe bedeutet, hergestellt.

Verfahren (c): Das Verfahren (c) betrifft vor allen Dingen den Austausch der Halogenreste Chlor, Brom oder Iod durch nukleophile Substitution mit einem nukleophilen Fluorierungsmittel. Zu nennen sind hier z.B. Kaliumfluorid, KHF₂ oder Tetrabutylammoniumfluorid [s. z.B. J. Org. Chem. 49, 3216 (1984)].

Die Ausgangsverbindungen der Formel IIIa können z.B. durch Halogenierung von Verbindungen der Formel II, etwa mit N-Halogensuccinimid, z.B. N-Brom- oder N-Chlorsuccinimid, einem Sulfurylhalogenid, z.B. SO₂Cl₂, oder elementarem Halogen, z.B. Cl₂ oder Br₂, hergestellt werden.

Ferner ist es z.B. möglich, die Ausgangsverbindungen der Formel IIIa durch Halogenierung von Verbindungen der Formel III herzustellen, etwa durch Umsetzung mit einem Thionylhalogenid, z.B. SOCl₂ oder SOBr₂, einem Phosphorhalogenid, z.B. PBr₃, PI₃ oder PCl₅, oder einer Halogenwasserstoffsäure, z.B. HBr.

Verfahren (d): In einer Verbindung der Formel IV steht eine in den Rest X überführbare Gruppe X' z.B. für Halogen oder Amino.

X' = Halogen, insbesondere Brom, kann z.B. durch Umsetzung mit einem Cyanierungsmittel, z.B. Kupfer(I)cyanid, in Cyano überführt werden. X' = Amino kann z.B. via Diazotierung beispielsweise in Cyano umgewandelt werden. Die Umwandlung von Substituenten an aromatischen Systemen gemäss Verfahren (d) ist an sich bekannt.

Für den Fall, dass in einer Verbindung der Formel IV die Gruppe R einen mit X' identischen Substituenten enthält, z.B. bei R = -C₆H₄-X', kann dieser bei der Durchführung des Verfahrens (d) gleichzeitig mit umgewandelt werden.

Die Ausgangsverbindungen der Formel IV werden z.B. analog Verfahren (a), (b) oder (c) hergestellt, wobei in den entsprechenden Umsetzungen anstelle des Restes X ein Rest X' eingesetzt wird.

Verbindungen der Formel I können in andere Verbindungen der Formel I umgewandelt werden.

Verfahrensgemäss erhältliche freie Verbindungen der Formel I mit salzbildenden Eigenschaften können in an sich bekannter Weise in ihre Salze überführt werden, Verbindungen mit basischen Eigenschaften z.B. durch Behandeln mit Säuren oder geeigneten Derivaten davon, Verbindungen mit sauren Eigenschaften z.B. durch Behandeln mit Basen oder geeigneten Derivaten davon.

Erfindungsgemäss erhältliche Gemische von Isomeren können in an sich bekannter Weise in die einzelnen Isomeren aufgetrennt werden, Racemate z.B. durch Bilden von Salzen mit optisch reinen salzbildenden Reagentien und Auftrennen des so erhältlichen Diastereomerengemisches, z.B. mittels fraktionierter Kristallisation.

Die oben angeführten Reaktionen können unter an sich bekannten Reaktionsbedingungen durchgeführt werden, in Ab- oder üblicherweise Anwesenheit von Lösungs- oder Verdünnungsmitteln, vorzugsweise solchen, die gegenüber den verwendeten Reagenzien inert sind und diese lösen, in Ab- oder Anwesenheit von Katalysatoren, Kondensationsmitteln oder neutralisierenden Agentien, je nach Art der Reaktion und/oder der Reaktionsteilnehmer bei erniedrigter, normaler oder erhöhter Temperatur, z.B. im Temperaturbereich von etwa -80°C bis etwa 200°C, vorzugsweise von etwa -78°C bis etwa 150°C, insbesondere von etwa -20°C bis etwa 150°C, z.B. beim Siedepunkt des verwendeten Lösungsmittels, unter atmosphärischem Druck oder in einem geschlossenen Gefäss, gegebenenfalls unter Druck, und/oder in einer inerten Atmosphäre, z.B. unter einer Stickstoffatmosphäre.

Infolge der engen Beziehung zwischen den Verbindungen der Formel I in freier Form und in Form von Salzen sind vor- und nachstehend unter den freien Verbindungen bzw. ihren Salzen sinn- und zweckmässig gegebenenfalls auch die entsprechenden Salze bzw. freien Verbindungen zu verstehen.

Die Verbindungen, einschliesslich ihrer Salze, können auch in Form von Hydraten erhalten werden, oder ihre Kristalle können z.B. das zur Kristallisation verwendete Lösungsmittel einschliessen.

Im Verfahren der vorliegenden Erfindung werden vorzugsweise solche Ausgangsstoffe eingesetzt, die zu den eingangs als besonders wertvoll beschriebenen Verbindungen führen.

Die Erfindung betrifft auch diejenigen Ausführungsformen des Verfahrens, bei denen man von einer auf einer beliebigen Verfahrensstufe als Zwischenprodukt erhältlichen Verbindung ausgeht und die fehlenden Verfahrensschritte durchführt, oder bei denen ein Ausgangsstoff unter den Reaktionsbedingungen gebildet oder in Form eines Derivates, z.B. eines Salzes davon, verwendet wird.

Die vorliegende Erfindung betrifft ebenfalls pharmazeutische Präparate, die als Wirkstoff eine der pharmakologisch wirksamen Verbindungen der Formel I enthalten. Besonders bevorzugt sind Präparate zur enteralen, insbesondere oralen, sowie zur parenterelen Verabreichung. Die Präparate enthalten den Wirkstoff allein oder vorzugsweise zusammen mit einem pharmazeutisch anwendbaren Trägermaterial. Die Dosierung des Wirkstoffs hängt von der zu behandelnden Krankheit, sowie von Spezies, deren Alter, Gewicht und individuellem Zustand, sowie von der Applikationsweise ab.

Die pharmazeutischen Präparate enthalten von etwa 0,1 % bis etwa 95 % des Wirkstoffs, wobei einzeldosierte Applikationsformen vorzugsweise von etwa 1 % bis etwa 90 % und nicht-einzeldosierte Applikationsformen vorzugsweise etwa 0,1 % bis etwa 20 % Wirkstoff aufweisen. Dosiseinheitsformen, wie Dragées, Tabletten oder Kapseln, enthalten von etwa 0,5 mg bis etwa 100 mg des Wirkstoffs.

Die pharmazeutischen Präparate der vorliegenden Erfindung werden in an sich bekannter Weise, z.B. mittels konventioneller Misch-, Granulier-, Dragier-, Lösungs-, oder Lyophilisierungsverfahren hergestellt. So kann man pharmazeutische Zusammensetzungen zur oralen Anwendung erhalten, indem man den Wirkstoff mit einem oder mehreren festen Trägerstoffen kombiniert, ein erhaltenes Gemisch gegebenenfalls granuliert, und das Gemisch bzw. Granulat, wenn erwünscht, gegebenenfalls durch Zugabe von zusätzlichen Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärken, z.B. Mais-, Weizen-, Reis- oder Kartoffelstärke, Methylcellulose, Hydroxypropylmethylcellulose, Natriumcarboxymethylcellulose und/oder Polyvinylpyrrolidon, und/oder, wenn erwünscht, Sprengmittel, wie die obengenannten Stärken, ferner Carboxymethylstärke, quervernetztes Polyvinylpyrrolidon, Alginsäure oder ein Salz davon, wie Natriumalginat. Zusätzliche Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol, oder Derivate davon.

Dragée-Kerne können mit geeigneten, gegebenenfalls Magensaft-resistenten Ueberzügen versehen werden, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Oral anwendbare pharmazeutische Zusammensetzungen sind ebenfalls Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Maisstärke, Bindemitteln und/oder Gleitmitteln, wie Talk oder Magnesiumstearat, und gegebenenfalls von Stabilisatoren, enthalten. In weichen Kapseln ist der Wirkstoff vorzugsweise in geeigneten flüssigen Hilfsstoffen, wie fetten Oelen, Paraffinöl oder flüssigen Polyethylenglykolen, gelöst oder suspendiert, wobei ebenfalls Stabilisatoren zugefügt sein können.

Weitere orale Applikationsformen sind z.B. in üblicher Weise bereitete Sirups, die den Wirkstoff z.B. in suspendierter Form und in einer Konzentration von ca. 0,01 % bis 2 %, vorzugsweise ca. 0,1 % oder in einer ähnlichen Konzentration, die z.B. beim Abmessen von 5 oder 10 ml eine geeignete Einzeldosis ergibt, enthalten. Ferner kommen z.B. auch pulverförmige oder flüssige Konzentrate zur Bereitung von Shakes, z.B. in Milch, in Betracht. Solche Konzentrate können auch in Einzeldosismengen abgepackt sein.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole oder höhere Alkanole.

Zu parenteralen Verabreichung eignen sich in erster Linie wässrige Lösungen eines Wirkstoffes in wasserlöslicher Form, z.B. eines wasserlöslichen Salzes, oder wässrige Injektionssuspensionen, welche viskositätserhöhende Stoffe, z.B. Natriumcarboxymethylcellulose, Sorbit und/oder Dextran und gegebenenfalls Stabilisatoren enthalten. Dabei kann der Wirkstoff, gegebenenfalls zusammen mit Hilfsstoffen, auch in Form eines Lyophilisats vorliegen und vor der parenteralen Verabreichung durch Zugabe von geeigneten Lösungsmitteln in Lösung gebracht werden.

Lösungen, wie sie z.B. für die parenterale Verabreichung verwendet werden, können auch als Infusionslösungen angewandt werden.

Möglich ist ein Verfahren zur Behandlung der oben genannten, insbesondere östrogenabhängigen, Krankheitszustände, beispielsweise von einem an einer derartigen Erkrankung leidenden Säugetier, z. B. Menschen, der dieser Behandlung bedarf, beispielsweise durch Verabreichung einer therapeutisch gegen die genannten Erkrankungen wirksame Menge einer der erfindungsgemässen Verbindungen an das Säugetier. Die Verbindungen der vorliegenden Erfindung können prophylaktisch oder therapeutisch verabreicht werden, wobei man sie vorzugsweise in Form von pharmazeutischen Präparaten verwendet. Dabei wird bei einem Körpergewicht von etwa 70 kg eine tägliche Dosis von etwa 0,5 mg bis etwa 100 mg, vorzugsweise von etwa 1 mg bis etwa 20 mg, einer Verbindung der vorliegenden Erfindung verabreicht.

Die nachfolgenden Beispiele illustrieren die vorliegende Erfindung; Temperaturen werden in Grad Celsius angegeben. Folgende Abkürzungen werden verwendet: Ether = Diethylether; Ethylacetat = Essigsäureethylester; THF = Tetrahydrofuran; Hexan = n-Hexan; DMSO = Dimethylsulfoxid; DMF = Dimethylformamid; N-Fluor-dimethylsaccharinsultam = N-Fluor-3,3-dimethyl-2,3-dihydro-1,2-benzothiazol-1,1-dioxid; DC = Dünnschichtchromatographie; RT = Raumtemperatur; MS(FAB) = Massenspektrum ("Fast Atom Bombardment").

### Beispiel 1: 4-[α-(4-Cyanophenyl)-α-fluor-1-(1,2,4-triazolyl)methyl]-benzonitril

Eine Losung von 0,8 mmol Kaliumhexamethyldisilazan in 1,6 ml Toluol wird mit 5 ml THF verdünnt und nach Abkühlen auf -78° mit einer Losung von 190 mg 4-[α-(4-Cyanophenyl)-1-(1,2,4-triazolyl)methyl]-benzonitril (s. EP-A-236 940, Bsp. 20a) in 3 ml THF versetzt. Nach 1 h Rühren bei gleicher Temperatur wird zur dunkelroten Lösung 301 mg N-Fluor-dimethylsaccharinsultam in 3 ml THF getropft. Nach weiteren 1,5 h bei -78° wird das Reaktionsgemisch innerhalb 1 h auf RT erwärmt und auf eine gesättigte Lösung von Ammoniumchlorid in Wasser gegossen und anschliessend mit Methylenchlorid extrahiert. Trocknen über Magnesiumchlorid und Eindampfen des Lösungsmittels ergibt das Rohprodukt, welches mittels Flashchromatographie (SiO₂, Hexan/Ethylacetat 9:1,4:1 bis 1:1) gereinigt wird. DC (SiO₂, CHCl₃/Methanol 9:1, Rf = 0,85); IR (KBr) : 2220 cm⁻¹; ¹H-NMR (CDCl₃): δ (ppm) = 7,46 und 7,76 (8H,m), 8,07 (1H,s), 8,16 (1H,s).

### Beispiel 2: 4-[α-(4-Cyanophenyl)-α-fluor-(2-tetrazolyl)methyl]-benzonitril

Analog Beispiel 1 wird 4-[α-(4-Cyanophenyl)-(2-tetrazolyl)methyl]-benzonitril (s. EP-A-408 509, Bsp. 7 und 2) mit N-Fluor-dimethylsaccharinsultam in die Titelverbindung überführt; Smp. 145-146°.

### Beispiel 3: 4-[α-(4-Cyanophenyl)-α-fluor-(1-tetrazolyl)methyl]-benzonitril

Analog Beispiel 1 wird 4-[α-(4-Cyanophenyl)-(1-tetrazolyl)methyl]-benzonitril (s. EP-A-408 509, Bsp. 7) mit N-Fluor-dimethylsaccharinsultam in die Titelverbindung überführt.

### Beispiel 4: 4-[α-(4-Cyanophenyl)-α-fluor-(1-imidazolyl)methyl]-benzonitril

Analog Beispiel 1 werden 1,075 g 4-[α-(4-Cyanophenyl)-(1-imidazolyl)methyl]-benzonitril (s. EP-A-236 940, Bsp. 2a, 3, 4 und 23) mit 930 mg Kaliumhexamethyldisilazan und 1,7 g N-Fluor-dimethylsaccharinsultam in die Titelverbindung überführt; Smp. 133°, MS(FAB): (M+H)⁺ = 303, DC (Methylenchlorid/Methanol 9:1): Rf = 0,7.

### Beispiel 5: 4-[α-(4-Cyanophenyl)-α-fluor-1-(1,2,3-triazolyl)methyl]-benzonitril

Analog Beispiel 1 wird 4-[α-(4-Cyanophenyl)-1-(1,2,3-triazolyl)methyl]-benzonitril mit N-Fluor-dimethylsaccharinsultam in die Titelverbindung überführt; Smp. 138-140°.

Die Ausgangsverbindung wird wie folgt hergestellt:

### (a) 4-[α-(4-Cyanophenyl)-1-(1,2,3-triazolyl)methyl]-benzonitril

Eine Lösung von 640 mg 4-[1-(1,2,3-Triazolyl)methyl]-benzonitril in 5 ml DMF wird über 30 min bei konstanter Temperatur (25-26,5°) zu einem Gemisch von 1,07 g Kalium-tert.-butoxid in 5 ml DMF getropft. Nach weiteren 30 min bei 20° versetzt man das Reaktionsgemisch mit einer Lösung von 525 mg 4-Fluorbenzonitril in 5 ml DMF und rührt 1,5 h bei Raumtemperatur. Dann wird auf 0° abgekühlt mit CH₂Cl₂ verdünnt und mit 6N HCl neutralisiert. Man engt das Reaktionsgemisch ein, nimmt es in Wasser/CH₂Cl₂ auf und trennt die wässrige Phase ab. Die organische Phase wird mit Sole gewaschen, über Natriumsulfat getrocknet und eingeengt. Das Rohprodukt wird durch Säulenchromatographie gereinigt (SiO₂, Toluol bis Toluol/Ethylacetat 3:1) und aus CH₂Cl₂/Ethanol/Hexan kristallisiert. Man erhält so die Ausgangsverbindung (a), Smp. >230°; IR (CH₂Cl₂): 2230, 1605, 1500, 1160 cm⁻¹.

Die Vorstufe zur Herstellung der Ausgangsverbindung (a) wird wie folgt hergestellt:

### (1) 4-[1-(1,2,3-Triazolyl)methyl]-benzonitril

Eine Lösung von 15,13 g 4-Brommethylbenzonitril in 375 ml Aceton wird nacheinander mit 8 g 1,2,3-Triazol, 10,67 g Kaliumcarbonat und 750 mg Kaliumiodid versetzt. Das Reaktionsgemisch wird dann 7,5 h bei 55° gerührt, danach abgekühlt und eingeengt. Der Rückstand wird in CH₂Cl₂ gelöst und nacheinander mit Wasser und Sole gewaschen. Nach dem Trocknen über Natriumsulfat wird die Losung eingeengt und das erhaltene Rohprodukt durch Säulenchromatographie gereinigt (SiO₂, Toluol/Ethylacetat 3:19). Man erhält so die Vorstufe (1), IR (CH₂Cl₂): 2230, 1615, 1225, 1075 cm⁻¹.

### Beispiel 6: 4-[α-(4-Bromphenyl)-α-fluor-1-(1,2,4-triazolyl)methyl]-benzonitril

Analog Beispiel 1 wird 4-[α-(4-Bromphenyl)-1-(1,2,4-triazolyl)methyl]-benzonitril mit N-Fluor-dimethylsaccharinsultam in die Titelverbindung überführt.

Die Ausgangsverbindung wird wie folgt hergestellt:

### (a) 4-[α-(4-Bromphenyl)-1-(1,2,4-triazolyl)methyl]-benzonitril

Analog Beispiel 5(a) werden 190 mg 1-(4-Brombenzyl)-1,2,4-triazol mit 188 mg Kalium-tert.-butoxid und 106 mg 4-Fluorbenzonitril in die Ausgangsverbindung (a) überführt; ¹H-NMR (CDCl₃): δ = 6,73 (1H,s), 7,05 und 7.55 (4H,m), 7,2 und 7,68 (4H,m), 8,02 (1H,s), 8,05 (1H,s).

Die Vorstufe zur Herstellung der Ausgangsverbindung (a) wird wie folgt hergestellt:

### (1) 1-(4-Brombenzyl)-1,2,4-triazol

Ein Gemisch von 1 g 4-Brombenzylbromid, 0,41 g 1,2,4-Triazol, 0,55 g Kaliumcarbonat und 33 mg Kaliumiodid in 30 ml Aceton wird 20 h bei 50° gerührt. Der Feststoff wird abfiltriert und die Lösung eingedampft. Die erhaltene rohe Vorstufe (1)wird durch Säulenchromatographie (SiO₂, Hexan/Ethylacetat 1:1) gereinigt und aus Ether kristallisiert; Smp. 77-79°; ¹H-NMR (CDCl₃): δ = 5,3 (2H,s), 7,15 und 7,5 (4H,m), 7,95 (1H,s), 8,08 (1H,m).

### Beispiel 7: 4-[α-(4-Cyanophenyl)-α-fluor-1-(1,2,4-triazolyl)methyl]-benzonitril

10 ml THF werden auf -30° gekühlt. Es werden erst 0,17 ml (1,2 mmol) Diisopropylamin und dann 0,75 ml (1,2 mmol) einer 1,6 M Lösung von n-Butyllithium in Hexan zugegeben und auf -70° gekühlt. Man tropft 285 mg (1 mmol) 4-[α-(4-Cyanophenyl)-1-(1,2,4-triazolyl)methyl]-benzonitril (s. EP-A-236 940, 20a), gelöst in 4 ml THF, langsam zu und rührt 3 h bei -70°. Dann werden 346,8 mg (1,2 mmol) N-Fluor-2,4,6-trimethylpyridinium-trifluormethylsulfonat zugegeben, worauf sich die zuvor dunkelrot gefärbte Lösung langsam entfärbt. Man lässt das Reaktionsgemisch auf RT erwärmen, giesst es auf eine gesättigte wässrige Ammoniumchloridlösung und extrahiert mit Methylenchlorid. Die organischen Extrakte werden über Magnesiumchlorid getrocknet und eingedampft, wobei man das Rohprodukt erhält, welches durch Flashchromatographie (SiO₂, Hexan/Ethylacetat 9:1, 4:1 bis 1:1) gereinigt wird. DC (SiO₂, CHCl₃/Methanol 9:1): Rf = 0,85; IR (KBr): 2220 cm⁻¹; ¹H-NMR (CDCl₃): δ = 7,46 und 7,76 (8H, m); 8,07 (1H, s), 8,16 (1H, s).

### Beispiel 8: 4-[α-(4-Cyanophenyl)-α-fluor-(2-tetrazolyl)methyl]-benzonitril

399 mg Kaliumhexamethyldisilazan werden bei -5° in 4 ml abs. Toluol gelöst und mit 12 ml abs. THF verdünnt. Man kühlt diese Lösung auf -75° ab und versetzt sie tropfenweise innerhalb von 10 min mit einer Lösung von 475 mg 4-[α-(4-Cyanophenyl)-(2-tetrazolyl)-methyl]-benzonitril (s. EP-A-408 509, Bsp. 7 und 2) in 7,5 ml abs. THF. Das dunkelrote Reaktionsgemisch wird noch 1 h bei der gleichen Temperatur gerührt und dann innerhalb von 15 min mit einer Lösung von 0,75 g N-Fluor-dimethylsaccharinsultam in 7,5 ml abs. THF versetzt, weitere 1,5 h gerührt und dann innerhalb von 1 h auf RT erwärmt. Die Lösung wird auf 50 ml einer gesättigten, wässrigen NaCl-Lösung gegossen und mit Methylenchlorid extrahiert. Die organische Phase wird mit Sole gewaschen und nach Trocknung über Natriumsulfat eingeengt. Das erhaltene Rohprodukt wird dreimal mit Ether verrührt, durch Säulenchromatographie (Kieselgel, Ethylacetat/Hexan 1:1) gereinigt und aus Hexan kristallisiert; Smp. 145-146°, MS(FAB): (M+H)⁺ = 305, DC (Ethylacetat/Hexan 1:1): Rf = 0,5.

### Beispiel 9: 4-[α-(4-Cyanophenyl)-α-fluor-1-(1,2,3-triazolyl)methyl]-benzonitril

Eine auf -5° gekühlte Lösung von 798 mg Kaliumhexamethyldisilazan in 8 ml abs. Toluol wird mit 25 ml abs. THF verdünnt, auf -75° gekühlt und innerhalb von 15 min mit einer Lösung von 950 mg 4-[α-(4-Cyanophenyl)-1-(1,2,3-triazolyl)methyl]-benzonitril (Bsp. 5a) in 15 ml abs. THF und 1 ml abs. DMF versetzt. Nach 1 h Rühren bei -75° wird eine Lösung von 1,5 g N-Fluor-dimethylsaccharinsultam in 15 ml THF zugegeben. Nach weiteren 1,5 h Rühren wird das Kühlbad entfernt, wonach sich das Reaktionsgemisch innerhalb 1 h auf RT erwärmt. Das Reaktionsgemisch wird auf 100 ml einer gesättigten, wässrigen Ammoniumchlorid-Lösung gegossen und mit Methylenchlorid extrahiert. Die organische Phase wird mit Sole gewaschen, über Natriumsulfat getrocknet und eingeengt. Das erhaltene Rohprodukt wird zweimal mit Ether verrührt und durch Säulenchromatographie (Kieselgel, Ethylacetat/Hexan 1:1) gereinigt; Smp. 138-140°, MS(FAB): (M+H)⁺ = 304, DC (Ethylacetat/Hexan 1:1): Rf = 0,41.

### Beispiel 10: Es werden 10000 Tabletten hergestellt, die je 5 mg Wirkstoff, z.B. eine der in den Beispielen 1-9 hergestellten Verbindungen, enthalten:

| Zusammensetzung: | |
|---|---|
| Wirkstoff | 50,00 g |
| Milchzucker | 2535,00 g |
| Maisstärke | 125,00 g |
| Polyethylenglykol 6.000 | 150,00 g |
| Magnesiumstearat | 40,00 g |
| Gereinigtes Wasser | quantum satis |

Verfahren: Alle pulverigen Bestandteile werden durch ein Sieb mit einer Maschenweite von 0,6 mm gesiebt. Dann wird der Wirkstoff, der Milchzucker, das Magnesiumstearat und die Hälfte der Stärke in einem geeigneten Mischer vermischt. Die andere Hälfte der Stärke wird in 65 ml Wasser suspendiert und die entstandene Suspension zu einer siedenden Lösung des Polyethylenglykols in 260 ml Wasser gegeben. Die gebildete Paste wird dem Pulvergemisch zugesetzt und gegebenenfalls unter Zugabe von mehr Wasser granuliert. Das Granulat wird über Nacht bei 35°C getrocknet, durch ein Sieb mit 1,2 mm Maschenweite getrieben und zu Tabletten, welche eine Bruchrille aufweisen, gepresst.

### Beispiel 11: Es werden 1000 Kapseln hergestellt, die je 10 mg Wirkstoff, z.B. eine der in den Beispielen 1-9 hergestellten Verbindungen, enthalten:

| Zusammensetzung: | |
|---|---|
| Wirkstoff | 10,00 g |
| Milchzucker | 207,00 g |
| Modifizierte Stärke | 80,00 g |
| Magnesiumstearat | 3,00 g |

Verfahren: Alle pulverigen Bestandteile werden durch ein Sieb mit einer Maschenweite von 0,6 mm gesiebt. Dann wird der Wirkstoff in einem geeigneten Mischer zuerst mit dem Magnesiumstearat und dann mit dem Milchzucker und der Stärke bis zur Homogenität vermischt. Hartgelatinekapseln Nr. 2 werden mit je 300 mg der erhaltenen Mischung unter Verwendung einer Kapselfüllmaschine gefüllt.

## Patentansprüche (Patentansprüche für folgende(n) Vertragsstaat(en): BE, DK, DE, ES, FR, GB, IT, NL, AT, SE, CH, LI, LU)

1. Verbindungen der Formel I, 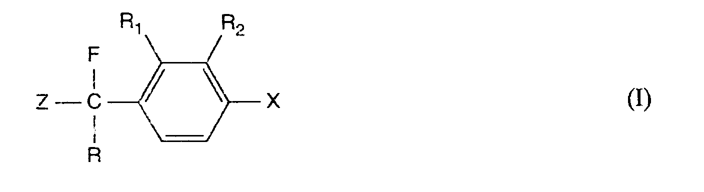 worin Z 1-Imidazolyl, 1-(1,2,4-Triazolyl), 1-(1,3,4-Triazolyl), 1-(1,2,3-Triazolyl), 1-Tetrazolyl oder 2-Tetrazolyl bedeutet; R₁ und R₂ unabhängig voneinander Wasserstoff oder Niederalkyl bedeuten; oder R₁ und R₂ zusammen für 1,4-Butylen oder eine Benzogruppe stehen; R für 4-Cyanophenyl oder 4-Bromophenyl steht; und X Cyano bedeutet; und Salze davon; wobei das Präfix "Nieder" einen Rest bis und mit höchstens 7 Kohlenstoffatomen bezeichnet.

2. Verbindungen der Formel I gemäss Anspruch 1, worin Z 1-Imidazolyl, 1-(1,2,4-Triazolyl), 1-(1,2,3-Triazolyl), 1-Tetrazolyl oder 2-Tetrazolyl bedeutet; R₁ und R₂ für Wasserstoff stehen; R 4-Cyanophenyl oder 4-Bromophenyl bedeutet; und X für Cyano steht; und pharmazeutisch verwendbare Salze davon.

3. 4-[α-(4-Cyanophenyl)-α-fluor-1-(1,2,4-triazolyl)methyl]-benzonitril gemäss Anspruch 1 oder pharmazeutisch verwendbare Salze davon.

4. 4-[α-(4-Cyanophenyl)-α-fluor-1-(1,2,3-triazolyl)methyl]-benzonitril gemäss Anspruch 1 oder pharmazeutisch verwendbare Salze davon.

5. Pharmazeutische Präparate enthaltend eine Verbindung gemäss einem der Ansprüche 1 bis 4 und mindestens ein pharmazeutisch verwendbares Trägermaterial.

6. Eine Verbindung gemäss einem der Ansprüche 1 bis 4 zur Anwendung in einem Verfahren zur therapeutischen Behandlung des tierischen oder menschlichen Körpers.

7. Verwendung einer Verbindung gemäss einem der Ansprüche 1 bis 4 zur Herstellung pharmazeutischer Präparate für die Behandlung von Krankheiten, die auf eine Hemmung des Enzyms Aromatase ansprechen.

8. Verfahren zur Herstellung einer Verbindung der Formel I gemäss Anspruch 1, dadurch gekennzeichnet, dass man
(a) eine Verbindung der Formel II, 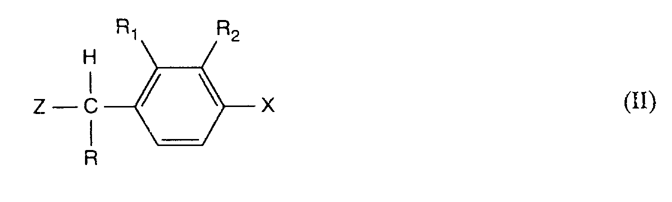 worin Z, R, R₁, R₂ und X wie unter Formel I definiert sind, mit einem Fluorierungsmittel umsetzt, oder
(b) zur Herstellung von Verbindungen der Formel I, worin Z über ein Ringkohlenstoffatom gebunden ist, eine Verbindung der Formel III, 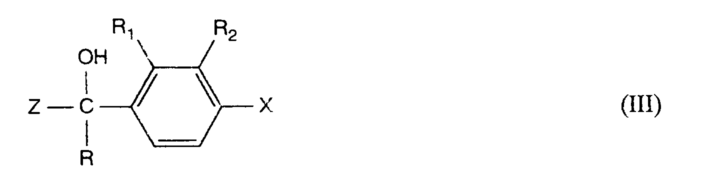 worin Z, R, R₁, R₂ und X wie unter Formel I definiert sind, mit einem Fluorierungsmittel umsetzt, oder
(c) eine Verbindung der Formel IIIa, 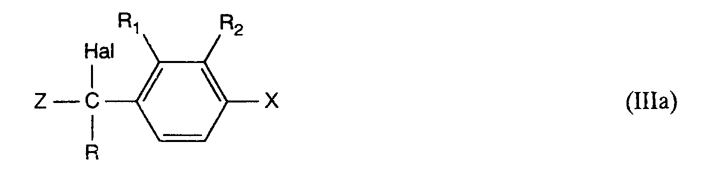 worin Hal Chlor, Brom oder Iod bedeutet und Z, R, R₁, R₂ und X wie unter Formel I definiert sind, mit einem Fluorierungsmittel umsetzt, oder
(d) eine Verbindung der Formel IV, 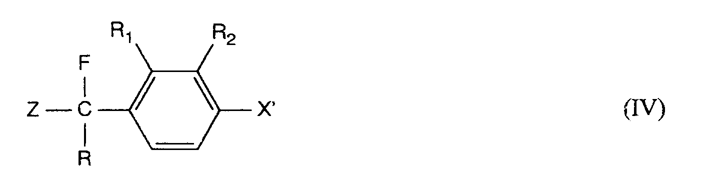 worin Z, R, R₁ und R₂ wie unter Formel I definiert sind und X' eine in einen Rest X überführbare Gruppe bedeutet, die Gruppe X' in den Rest X überführt, und/oder eine erhaltene Verbindung der Formel I in eine andere Verbindung der Formel I umwandelt, und/oder ein erhaltenes Salz in die freie Verbindung oder in ein anderes Salz umwandelt, und/oder eine erhaltene freie Verbindung der Formel I in ein Salz umwandelt, und/oder ein erhaltenes Gemisch von isomeren Verbindungen der Formel I in die einzelnen Isomeren auftrennt.

## Patentansprüche (Patentansprüche für folgende(n) Vertragsstaat(en): GR)

1. Verfahren zur Herstellung einer Verbindung der Formel I 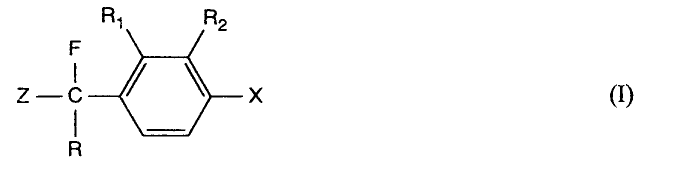 worin Z 1-Imidazolyl, 1-(1,2,4-Triazolyl), 1-(1,3,4-Triazolyl), 1-(1,2,3-Triazolyl), 1-Tetrazolyl oder 2-Tetrazolyl bedeutet; R₁ und R₂ unabhängig voneinander Wasserstoff oder Niederalkyl bedeuten; oder R₁ und R₂ zusammen für 1,4-Butylen oder eine Benzogruppe stehen; R für 4-Cyanophenyl oder 4-Bromophenyl steht; und X Cyano bedeutet; und Salzen davon; wobei das Präfix "Nieder" einen Rest bis und mit höchstens 7 Kohlenstoffatomen bezeichnet,
dadurch gekennzeichnet, dass man
(a) eine Verbindung der Formel II, 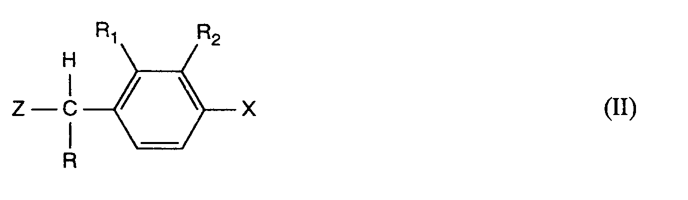 worin Z, R, R₁, R₂ und X wie unter Formel I definiert sind, mit einem Fluorierungsmittel umsetzt, oder
(b) zur Herstellung von Verbindungen der Formel I, worin Z über ein Ringkohlenstoffatom gebunden ist, eine Verbindung der Formel III, 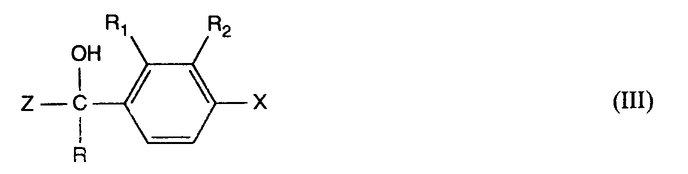 worin Z, R, R₁, R₂ und X wie unter Formel I definiert sind, mit einem Fluorierungsmittel umsetzt, oder
(c) eine Verbindung der Formel IIIa, 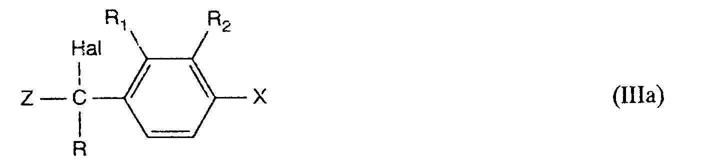 worin Hal Chlor, Brom oder Iod bedeutet und Z, R, R₁, R₂ und X wie unter Formel I definiert sind, mit einem Fluorierungsmittel umsetzt, oder
(d) eine Verbindung der Formel IV, 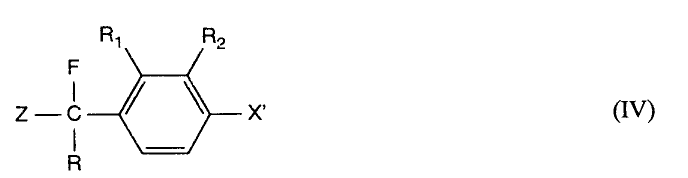 worin Z, R, R₁ und R₂ wie unter Formel I definiert sind und X' eine in einen Rest X überführbare Gruppe bedeutet, die Gruppe X' in den Rest X überführt, und/oder eine erhaltene Verbindung der Formel I in eine andere Verbindung der Formel I umwandelt, und/oder ein erhaltenes Salz in die freie Verbindung oder in ein anderes Salz umwandelt, und/oder eine erhaltene freie Verbindung der Formel I in ein Salz umwandelt, und/oder ein erhaltenes Gemisch von isomeren Verbindungen der Formel I in die einzelnen Isomeren auftrennt.

2. Verfahren gemäss Anspruch 1 zur Herstellung von Verbindungen der in Anspruch 1 gezeigten Formel 1, worin Z 1-Imidazolyl, 1-(1,2,4-Triazolyl), 1-(1,2,3-Triazolyl), 1-Tetrazolyl oder 2-Tetrazolyl bedeutet; R₁ und R₂ für Wasserstoff stehen; R 4-Cyanophenyl oder 4-Bromophenyl bedeutet; und X für Cyano steht; und pharmazeutisch verwendbaren Salzen davon, dadurch gekennzeicnet, dass man entsprechend substituierte Ausgangsmaterialien verwendet.

3. Verfahren gemäss Anspruch 1 zur Herstellung einer Verbindung der in Anspruch 1 gezeigten Formel I mit dem Namen 4-[α-(4-Cyanophenyl)-α-fluor-1-(1,2,4-triazolyl)-methyl]-benzo nitril oder von pharmazeutisch verwendbaren Salzen davon, dadurch gekennzeichnet, dass man entsprechend substituierte Ausgangsmaterialien verwendet.

4. Verfahren gemäss Anspruch 1 zur Herstellung einer Verbindung der in Anspruch 1 gezeigten Formel I mit dem Namen 4-[α-(4-Cyanophenyl)-α-fluor-1-(1,2,3-triazolyl)-methyl]-benzonitril oder von pharmazeutisch verwendbaren Salzen davon, dadurch gekennzeichnet, dass man entsprechend substituierte Ausgangsmaterialien verwendet.

5. Ein Verfahren zur Herstellung eines pharmazeutischen Präparates, dadurch gekennzeichnet, dass man eine gemäss einem der in den Ansprüchen 1 bis 4 genannten Verfahren erhaltene Verbindung der Formel I als Wirkstoff mit einem oder mehreren pharmazeutisch anwendbaren Trägermaterialien versetzt.

6. Ein Verfahren zur Herstellung eines pharmazeutischen Präparates, dadurch gekennzeichnet, dass man eine gemäss einem der in den Ansprüchen 1 bis 5 genannten Verfahren erhaltene Verbindung der Formel I als Wirkstoff mit einem oder mehreren pharmazeutisch anwendbaren Trägermaterialien versetzt, wobei der Wirkstoff im erhaltenen Präparat in einem Gewichtsanteil von 1 bis 90% vorliegt.

## Claims (Claims for the following Contracting State(s): BE, DK, DE, ES, FR, GB, IT, NL, AT, SE, CH, LI, LU)

1. A compound of formula I 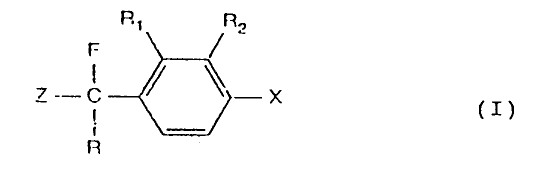 wherein Z is 1-imidazolyl, 1-(1,2,4-triazolyl), 1-(1,3,4-triazolyl), 1-(1,2,3-triazolyl), 1-tetrazolyl or 2-tetrazolyl; each of R₁ and R₂, independently of the other, is hydrogen or lower alkyl; or R₁ and R₂ together are 1,4-butylene or form a benzo group; R is 4-cyanophenyl or 4-bromophenyl; and X is cyano; or a salt thereof; the term "lower" denoting a radical having up to a maximum of 7 carbon atoms.

2. A compound of formula I according to claim 1, wherein Z is 1-imidazolyl, 1-(1,2,4-triazolyl), 1-(1,2,3-triazolyl), 1-tetrazolyl or 2-tetrazolyl; R₁ and R₂ are hydrogen; R is 4-cyanophenyl or 4-bromophenyl; and X is cyano; or a pharmaceutically acceptable salt thereof.

3. 4-[α-(4-Cyanophenyl)-α-fluoro-1-(1,2,4-triazolyl)methyl]-benzonitrile according to claim 1 or a pharmaceutically acceptable salt thereof.

4. 4-[α-(4-Cyanophenyl)-α-fluoro-1-(1,2,3-triazolyl)methyl]-benzonitrile according to claim 1 or a pharmaceutically acceptable salt thereof.

5. A pharmaceutical composition comprising a compound according to any one of claims 1 to 4 and at least one pharmaceutically acceptable carrier.

6. A compound according to any one of claims 1 to 4 for use in a method for the therapeutic treatment of the animal or human body.

7. The use of a compound according to any one of claims 1 to 4 for the preparation of pharmaceutical compositions for the treatment of diseases responsive to inhibition of the enzyme aromatase.

8. A process for the preparation of a compound of formula I according to claim 1, which comprises
(a) reacting a compound of formula II 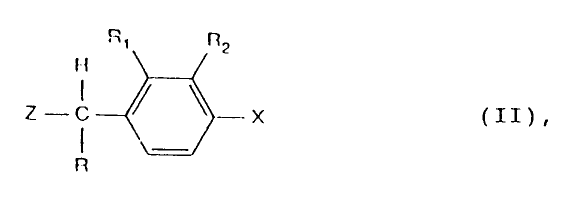 wherein Z, R, R₁, R₂ and X are as defined under formula I, with a fluorinating agent, or
(b) for the preparation of a compound of formula I wherein Z is bonded by way of a ring carbon atom, reacting a compound of formula III 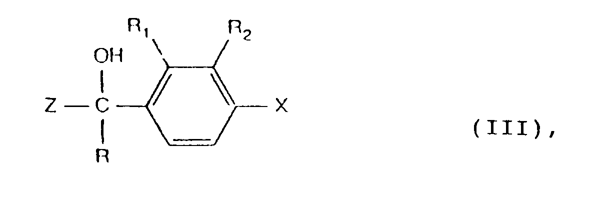 wherein Z, R, R₁, R₂ and X are as defined under formula I, with a fluorinating agent, or
(c) reacting a compound of formula IIIa 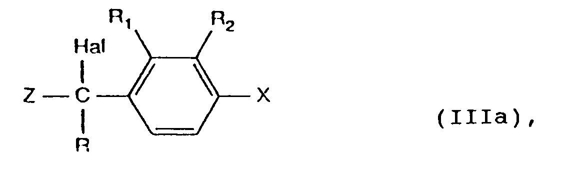 wherein Hal is chlorine, bromine or iodine, and Z, R, R₁, R₂ and X are as defined under formula I, with a fluorinating agent, or
(d) in a compound of formula IV 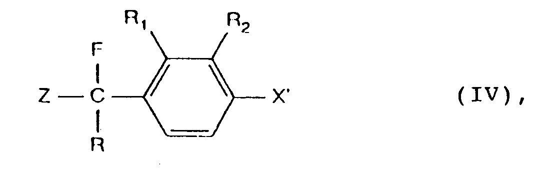 wherein Z, R, R₁ and R₂ are as defined under formula I and X' is a group that can be converted into a radical X, converting the group X' into the radical X, and/or converting a resulting compound of formula I into a different compound of formula I, and/or converting a resulting salt into the free compound or into a different salt, and/or converting a resulting free compound of formula I into a salt, and/or separating a resulting mixture of isomeric compounds of formula I into the individual isomers.

## Claims (Claims for the following Contracting State(s): GR)

1. A process for the preparation of a compound of formula I 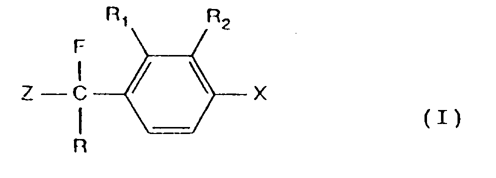 wherein Z is 1-imidazolyl, 1-(1,2,4-triazolyl), 1-(1,3,4-triazolyl), 1-(1,2,3-triazolyl), 1-tetrazolyl or 2-tetrazolyl; each of R₁ and R₂, independently of the other, is hydrogen or lower alkyl; or R₁ and R₂ together are 1,4-butylene or form a benzo group; R is 4-cyanophenyl or 4-bromophenyl; and X is cyano; or a salt thereof; the term "lower" denoting a radical having up to a maximum of 7 carbon atoms, which comprises
(a) reacting a compound of formula II 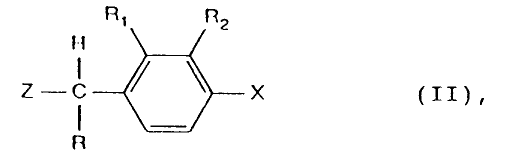 wherein Z, R, R₁, R₂ and X are as defined under formula I, with a fluorinating agent, or
(b) for the preparation of a compound of formula I wherein Z is bonded by way of a ring carbon atom, reacting a compound of formula III 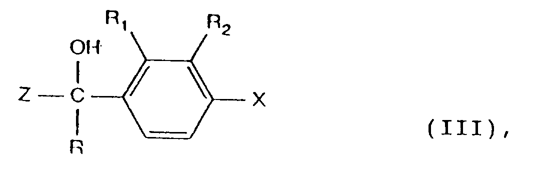 wherein Z, R, R₁, R₂ and X are as defined under formula I, with a fluorinating agent, or
(c) reacting a compound of formula IIIa 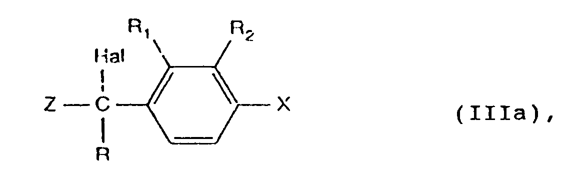 wherein Hal is chlorine, bromine or iodine, and Z, R, R₁, R₂ and X are as defined under formula I, with a fluorinating agent, or
(d) in a compound of formula IV 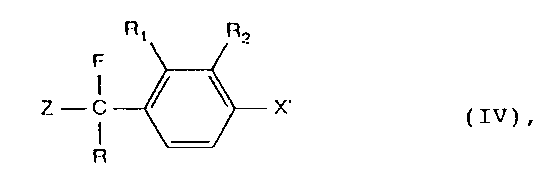 wherein Z, R, R₁ and R₂ are as defined under formula I and X' is a group that can he converted into a radical X, converting the group X' into the radical X, and/or converting a resulting compound of formula I into a different compound of formula I, and/or converting a resulting salt into the free compound or into a different salt, and/or converting a resulting free compound of formula I into a salt, and/or separating a resulting mixture of isomeric compounds of formula I into the individual isomers.

2. A process according to claim 1 for the preparation of a compound of formula I shown in claim 1, wherein Z is 1-imidazolyl, 1-(1,2,4-triazolyl), 1-(1,2,3-triazolyl), 1-tetrazolyl or 2-tetrazolyl; R₁ and R₂ are hydrogen; R is 4-cyanophenyl or 4-bromophenyl; and X is cyano; or a pharmaceutically acceptable salt thereof, which comprises using appropriately substituted starting materials.

3. A process according to claim 1 for the preparation of a compound of formula I shown in claim 1 having the name 4-[α-(4-cyanophenyl)-α-fluoro-1-(1,2,4-triazolyl)methyl]-benzonitrile, or a pharmaceutically acceptable salt thereof, which comprises using appropriately substituted starting materials.

4. A process according to claim 1 for the preparation of a compound of formula I shown in claim 1 having the name 4-[α-(4-cyanophenyl)-α-fluoro-1-(1,2,3-triazolyl)methyl]-benzonitrile, or a pharmaceutically acceptable salt thereof, which comprises using appropriately substituted starting materials.

5. A process for the preparation of a pharmaceutical composition, which comprises adding one or more pharmaceutically acceptable carriers to a compound of formula I as active ingredient obtained according to any one of the processes mentioned in claims 1 to 4.

6. A process for the preparation of a pharmaceutical composition, which comprises adding one or more pharmaceutically acceptable carriers to a compound of formula I as active ingredient obtained according to any one of the processes mentioned in claims 1 to 5, the active ingredient being present in the resulting composition in a proportion by weight of from 1 to 90 %.

## Revendications (Revendications pour l'(les) Etat(s) contractant(s) suivant(s): BE, DK, DE, ES, FR, GB, IT, NL, AT, SE, CH, LI, LU)

1. Composé de formule I 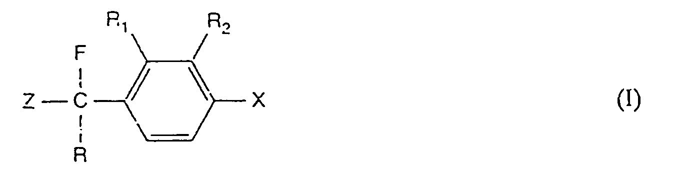 où Z représente le 1-imidazolyle, le 1-(1,2,4-triazolyle), le 1-(1,3,4-triazolyle), le 1-(1,2,3-triazolyle), le 1-tétrazolyle ou le 2-tétrazolyle ; R₁ et R₂ représentent, indépendamment l'un de l'autre, l'hydrogène ou un alkyle inférieur ; ou R₁ et R₂ représentent ensemble le 1,4-butylène ou un groupe benzo ; R représente le 4-cyanophényle ou le 4-bromophényle ; et X représente le cyano et leurs sels, le préfixe "inférieur" désignant un reste comportant au plus 7 atomes de carbone.

2. Composé de formule I selon la revendication 1, où Z représente le 1-imidazolyle, le 1-(1,2,4-triazolyle), le 1-(1,2,3-triazolyle), le 1-tétrazolyle ou le 2-tétrazolyle ; R₁ et R₂ représentent l'hydrogène ; R représente le 4-cyanophényle ou le 4-bromophényle ; et X représente le cyano ; et leurs sels pharmaceutiquement utilisables.

3. Le 4-[α-(4-cyanophényl-α-fluoro-1-(1,2,4-triazolyl)méthyl]-benzonitrile selon la revendication 1 ou ses sels pharmaceutiquement utilisables.

4. Le 4-[α-(4-cyanophényl-α-fluoro-1-(1,2,3-triazolyl)méthyl]-benzonitrile selon la revendication 1 ou ses sels pharmaceutiquement utilisables.

5. Préparations pharmaceutiques contenant un composé selon l'une des revendications 1 à 4 et au moins un support pharmaceutiquement utilisable.

6. Composé selon l'une des revendications 1 à 4 destiné à être utilisé dans un procédé de traitement thérapeutique du corps animal ou humain.

7. Utilisation d'un composé selon l'une des revendications 1 à 4 pour la fabrication de préparations pharmaceutiques destinées au traitement des maladies intéressées par une inhibition de l'enzyme aromatase.

8. Procédé pour la préparation d'un composé de formule I selon la revendication 1, caractérisé
(a) en ce que l'on fait réagir un composé de formule II 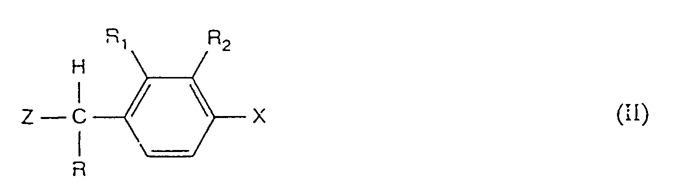 où Z, R, R₁, R₂ et X sont définis comme pour la formule I, avec un agent de fluoration ou
(b) en ce que l'on fait réagir, pour la préparation des composés de formule I où Z est lié par l'intermédiaire d'un atome de carbone cyclique, un composé de formule III 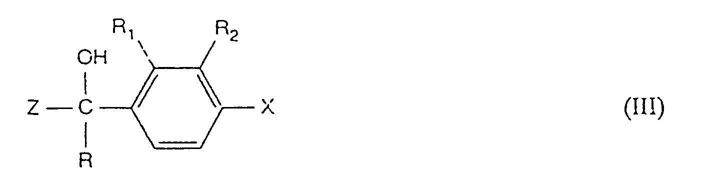 où Z, R, R₁, R₂ et X sont définis comme pour la formule I, avec un agent de fluoration ou
(c) en ce que l'on fait réagir un composé de formule IIIa 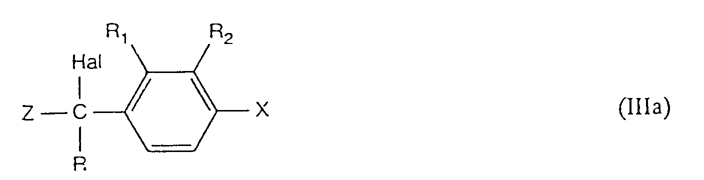 où Hal représente le chlore, le brome ou l'iode et Z, R, R₁, R₂ et X sont définis comme pour la formule I, avec un agent de fluoration ou
(d) en ce que l'on transforme dans un composé de formule IV 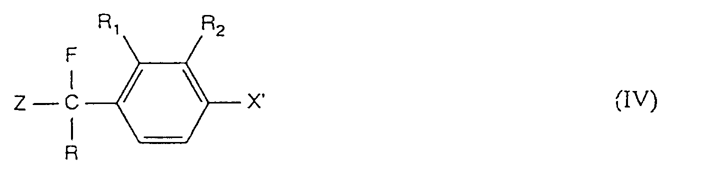 où Z, R, R₁ et R₂ sont définis comme pour la formule I et X' représente un groupe transformable en un reste X, le groupe X' en un reste X et/ou en ce que l'on transforme le composé obtenu de formule I en un autre composé de formule I et/ou le sel obtenu en un composé libre ou en un autre sel, et/ou le composé libre obtenu de formule I en un sel et/ou en ce que l'on sépare le mélange obtenu de composés isomères de formule I en les isomères individuels.

## Revendications (Revendications pour l'(les) Etat(s) contractant(s) suivant(s): GR)

1. Procédé pour la préparation d'un composé de formule I 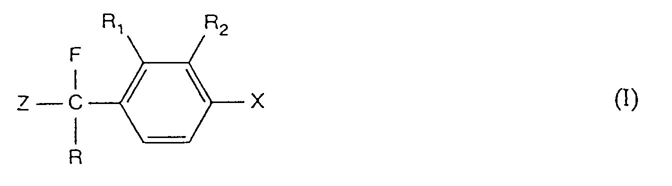 où Z représente le 1-imidazolyle, le 1-(1,2,4-triazolyle), le 1-(1,3,4-triazolyle), le 1-(1,2,3-triazolyle), le 1-tétrazolyle ou le 2-tétrazolyle ; R₁ et R₂ représentent, indépendamment l'un de l'autre, l'hydrogène ou un alkyle inférieur ; ou R₁ et R₂ représentent ensemble le 1,4-butylène ou un groupe benzo ; R représente le 4-cyanophényle ou le 4-bromophényle ; et X représente le cyano ; et leurs sels, le préfixe "inférieur" désignant un reste comportant au plus 7 atomes de carbone,
caractérisé
(a) en ce que l'on fait réagir un composé de formule II 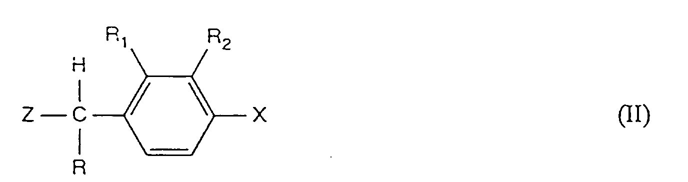 où Z, R, R₁, R₂ et X sont définis comme pour la formule I, avec un agent de fluoration ou
(b) en ce que l'on fait réagir, pour la préparation des composés de formule I où Z est lié par l'intermédiaire d'un atome de carbone cyclique, un composé de formule III 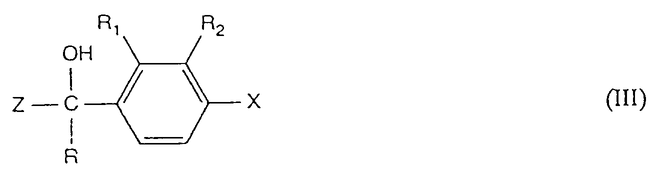 où Z, R, R₁, R₂ et X sont définis comme pour la formule I, avec un agent de fluoration ou
(c) en ce que l'on fait réagir un composé de formule IIIa 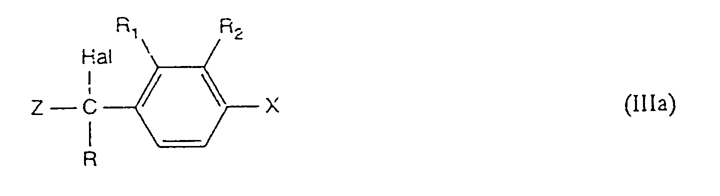 où Hal représente le chlore, le brome ou l'iode et Z, R, R₁, R₂ et X sont définis comme pour la formule I, avec un agent de fluoration ou
(d) en ce que l'on transforme dans un composé de formule IV 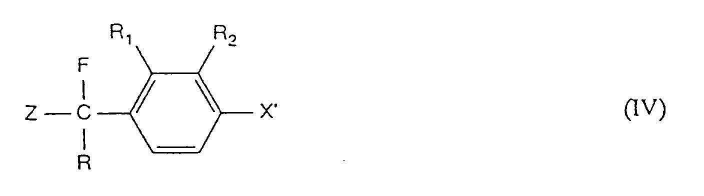 où Z, R, R₁ et R₂ sont définis comme pour la formule I et X' représente un groupe transformable en un reste X, le groupe X' en un reste X et/ou en ce que l'on transforme le composé obtenu de formule I en un autre composé de formule I et/ou le sel obtenu en un Composé libre ou en un autre sel, et/ou le composé libre obtenu de formule I en un sel et/ou en ce que l'on sépare le mélange obtenu de composés isomères de formule I en les isomères individuels.

2. Procédé selon la revendication 1 pour la préparation des composés de formule (I) représentée dans la revendication 1, où Z représente le 1-imidazolyle, le 1-(1,2,4-triazolyle), le 1-(1,2,3-triazolyle), le 1-tétrazolyle ou le 2-tétrazolyle ; R₁ et R₂ représentent l'hydrogène ; R représente le 4-cyanophényle ou le 4-bromophényle ; et X représente le cyano ; et leurs sels pharmaceutiquement utilisables, caractérisé en ce que l'on utilise des produits de départ substitués de façon correspondante.

3. Procédé selon la revendication 1 pour la préparation d'un composé de formule I représentée dans la revendication 1, dénommé 4-[α-(4-cyanophényl-α-fluoro-1-(1,2,4-triazolyl)méthyl]-benzonitrile ou de l'un de ses sels pharmaceutiquement utilisables, caractérisé en ce que l'on utilise des produits de départ substitués de façon correspondante.

4. Procédé selon la revendication 1 pour la préparation d'un composé de formule I représentée dans la revendication 1, dénommé 4-[α-(4-cyanophényl-α-fluoro-1-(1,2,3-triazolyl)méthyl]-benzonitrile ou l'un de ses sels pharmaceutiquement utilisables, caractérisé en ce que l'on utilise des produits de départ substitués de façon correspondante.

5. Un procédé pour la fabrication d'une préparation pharmaceutique, caractérisé en ce que l'on additionne un composé de formule I, obtenu par l'un des procédés décrits dans les revendications 1 à 4, utilisé comme substance active, d'un ou de plusieurs supports pharmaceutiquement utilisables.

6. Un procédé pour la fabrication d'une préparation pharmaceutique, caractérisé en ce que l'on additionne un composé de formule I obtenu par l'un des procédés décrits dans les revendications 1 à 5, utilisé comme substance active, d'un ou plusieurs supports pharmaceutiquement utilisables, la substance active étant présente dans la préparation obtenue dans une proportion en poids allant de 1 à 90 %.
